# EUROPEAN PATENT APPLICATION

(11) **EP 2 366 292 A1**
(43) Date of publication of application: **21.09.2011**
(21) Application number: 10156452.4
(22) Date of filing: 15.03.2010
(51) Int. Cl.: A23L 1/164, A23L 1/09, A23L 1/236, A23L 1/30, A23L 2/60

(54) **Artificial sweeteners and performance**

(71) Applicant: Nestec S.A., 1800 Vevey (CH)
(72) Inventor: Le Coutre, Johannes, 1009 Pully (CH); Stellingwerff, Trent, 1052 Le Mont-Sur-Lausanne (CH); Van Bladeren, Peter, 1802 Couseaux (CH); Damak, Sami, 1066 Epalignes (CH)
(74) Representative: Oldroyd, Richard Duncan

(57) **Abstract**

The present invention relates to the field of nutritional compositions. In particular, the present invention relates to a nutritional composition comprising sugar substitutes and to their use, e.g., to increase exogenous carbohydrate oxidation, which has been shown to increase performance, for example the performance of athletes.

## Description

The present invention relates to the field of nutritional compositions. In particular, the present invention relates to a nutritional composition comprising sugar substitutes and to their use, e.g., to increase exogenous carbohydrate oxidation, which has been shown to increase performance, for example the performance of athletes.

Carbohydrate (CHO) ingestion, for example, during exercise, has consistently been shown to improve endurance performance during both prolonged (>2 h) and short duration exercise (Jentjens RL and Jeukendrup AE, Br J Nutr 93: 485-492, 2005.).

However, a variety of symptoms may occur during exercise, which may be attributed to disorders of the upper (esophagus and stomach) or lower (small bowel and colon) gastrointestinal (Gl) tract. The prevalence of Gl disturbances is high (30 to 50% among endurance athletes) and seems to be related to CHO intake during exercise, especially running but also cycling, with the possibility that much of the CHO may just be sitting in the Gl tract, not being properly absorbed, and thus causing Gl distress (Brouns F, et al. Int J Sports Med 8: 175-189, 1987). Therefore, many athletes are faced with the decision of knowing that CHO intake during exercise can increase performance, but also may result in GI upset and disturbances, leading to a potential performance decrement.

To overcome this problem it was attempted to increase CHO absorption. In a series of recent studies, the laboratory of Jeukendrup et al. observed for example that a combination of glucose and fructose, or maltodextrin and fructose, can result in a significantly higher (20 to 40% higher) total exogenous CHO delivery during exercise compared to a single CHO source (ie. glucose alone) (Jeukendrup AE, Nutrition 20: 669-677, 2004, Jeukendrup AE and Jentjens R, Sports Med 29: 407-424, 2000).

The currently proposed mechanism underlying this effect is due to the fact that there are separate intestinal transporters for both glucose and fructose, namely SGLT-1 and GLUT-5, respectively. Recent published data (Currell K, Jeukendrup AE, Med Sci Sports Exerc 2008, 40: 275-281) also showed that this increased exogenous CHO oxidation from a glucose and fructose mixture translates into an 8% performance increase versus isocaloric glucose only beverages (and nearly 20% increase in performance versus water).

This is a huge and substantial increase in performance for endurance athletes.

For example, some PowerBar^{®} products now comprise this optimized multitransportable glucose: fructose 2:1 blend. Examples of these products are named C2MAX.

Very recently, high performance endurance athletes have started practicing low energy training. This is achieved by either over-night fasted morning exercise (De Bock K, et al., J Appl Physiol 2008, 104: 1045-1055) or by purposely training on low-muscle glycogen (Hansen AK, et al., J Appl Physiol 2005, 98: 93-99; Yeo WK, et al., J Appl Physiol 2008, 105: 1462-1470). It has been proposed that low calorie intake during endurance training may further enhance the training effect over time through the increase in fat oxidation enzymes, increase in mitochondrial content and an increase in fat oxidation during exercise Hansen AK, et al., J Appl Physiol 2005, 98: 93-99; Yeo WK, et al., J Appl Physiol 2008, 105: 1462-1470; De Bock K, et al., J Appl Physiol 2008, 104: 1045-1055). All of these are very desirable adaptations for endurance athletes, and thus allows endurance athlete to achieve a better performance during competition when energy supply is adequate and maximal (Hansen AK, et al., J Appl Physiol 2005, 98: 93-99).

However, although the carbohydrate absorption has been improved, for example by products such as C2MAX, and the incidence of Gl problems after carbohydrate uptake has been reduced during exercise, there still remains a need in the art to even further reduce the occurrence of Gl problems and to further improve CHO absorption. There also remains a need in the art to allow endurance athletes to train in low-energy conditions for optimal training adaptations, while still stimulating and up-regulating gut CHO transporters, for subsequent competition performance when CHO energy is provided. This would allow the endurance athletes to reap the full benefit of low-energy training, while not compromising their Gl tolerance to ingesting carbohydrates, which improve endurance performance. Finally, there was a need in the art to prime the Gl tract by acutely stimulating and up-regulating CHO transporters prior to exercise to allow an even further increased absorption and oxidation of carbohydrate. This is especially important during the initial stages of exercise when CHO absorption and oxidation are the lowest, and the biggest potential impact on muscle glycogen sparing (Stellingwerff T, et al., Pflugers Arch - Eur J Physiol, 2007, 454: 635-647).

In was the object of the present invention to improve the state of the art.

One object of the present invention was it to further reduce the occurrence of GI problems after CHO uptake, for example during exercise.

It was a further object of the present invention to further improve CHO absorption and oxidation, for example during exercise.

It was another object of the present invention to provide the art with a composition that allows it to increase CHO oxidation, which has been shown to increase performance.

It was a further object of the present invention to allow endurance athletes to train in low-energy conditions for optimal training adaptations, while still stimulating and up-regulating gut CHO transporters.

The present inventors were surprised to see that they could achieve these objects by the subject matter of the independent claims. The dependant claims further develop the idea of the present invention.

Surprisingly, even non-caloric sweeteners were found to have a performance increasing effect.

For example, a nutritional composition comprising a carbohydrate source containing glucose and fructose in a ratio in the range of 3:1 to 1:1, and/or at least one sugar substitute allowed achieving the desired effects.

Without wishing to be bound by theory, the present inventors believe that a high carbohydrate diet up-regulates the expression of the glucose transporter SGLT1 and glucose uptake by enterocytes. This up-regulation might be mediated by sweet taste receptors.

Sugar substitutes appear to be also effective in up-regulating intestinal SGLT1 expression and glucose uptake. This property of sugar substitutes appears to be linked to their perceived sweetness.

They also have the ability to cause an upregulation of GLUT-5 expression, a fructose transporter.

Therefore, the consumption of a diet rich in carbohydrates supplemented with sugar substitutes, e.g. during training, or during the immediate hours prior to competition, may improve glucose and/or fructose transport during exercise. Consequently, the body will be in a position to utilize the carbohydrates provided by a nutritional composition more effectively, while at the same time avoid the risk of Gl tract problems due to unabsorbed sugar in the gut. An increase in CHO oxidation will result, and performance and well-being is improved.

Optimising glucose and/or fructose transport appears to be necessary to increase performance. This may be achieved according to the present invention by the intake of sugars in combination with sugar substitutes before and/or during exercise.

For example, a composition such as a drink with high dose of sugar substitutes to be consumed during training and in the immediate hours prior to competition, will allow athletes to more efficiently benefit from the carbohydrates provided, for example by enhancing glucose and fructose transport and by minimising the unwanted effects of high amounts of sugar. This leads ultimately to an enhanced carbohydrate absorption, oxidation and increased performance.

The same can be achieved by the administration of a composition, such as a drink with an optimised mix of sugar and sugar substitutes to be taken during exercise and/or competition.

Hence, one embodiment of the present invention relates to a nutritional composition comprising glucose and fructose in a ratio in the range of 3:1 to 1:1, and at least one sugar substitute.

It was found that an intake of even 90 grams of carbohydrate per hour, compared to 30-60 g CHO/ h as officially recommended by the American College of Sports Medicine (ACSM), is well tolerable for the GI-tract if ingested as carbohydrate mixture comprising glucose and fructose in a ratio in the range of 3:1 to 1:1, preferably 2:1. For example, the carbohydrate mixture may also comprise glucose and fructose in a glucose-fructose ratio in the range of 3:1-2.3:1 or 1.7:1 to 1:1.

The ingestion of 90 grams/h of the above carbohydrate mixture will allow keeping the distress of the Gl-tract of athletes minimal, while resulting in 20-40% increase in CHO absorption and oxidation. Furthermore, it was found that the minimal Gl distress was not increased as compared with an intake of 60 grams of a carbohydrate mixture comprising glucose and fructose in a ratio in the range of 3.1 to 1:1, preferably 2:1 per hour.

The nutritional composition may be a food product, a drink, a food additive, or a nutraceutical, for example. It may also be provided in bar or in gel form.

It may be intended for humans or mammals, such as pets. In particular it is intended for individuals that need to perform, for example athletes, such as endurance athletes, or individuals in a test situation under pressure, but also for people that wish to perform better in their daily life such as at school or at work.

The compositions of the present invention may be intended to be consumed during exercise and/or briefly before or after exercise. Briefly before or after exercise corresponds to the time span from 1 hour before exercise to 1 hour after exercise.

A sugar substitute is a compound that duplicates the effect of sugar or corn syrup in taste, but has fewer calories. Preferably, compounds with little or no calories, such as non-caloric sweeteners, are used.

Fructose and glucose may be provided from a carbohydrate source. The carbohydrate source contains fructose and glucose in a digestible form. The carbohydrates may comprise or consist of monosaccharides, such as glucose or fructose as basic carbohydrate units. The monosaccharides may represent a part of disaccharides, such as sucrose, lactose, maltose or cellobiose. The monosaccharides such as glucose or fructose may also represent a part of oligosaccharides or polysaccharides. Preferred carbohydrate sources for the present invention are maltodextrins and/or dextrose.

The carbohydrate source may additionally comprise indigestible carbohydrates, in particular fibres.

Consequently, glucose and fructose may be provided as such, but may also be provided in the form of glucogenic compounds or fructogenic compounds.

A glucogenic compound is any compound, for example a polysaccharide that comprises a glucose unit. Glucose may then be provided from a glucogenic compound by enzymatic activity in the body, for example.

A fructogenic compound is any compound, for example a polysaccharide that comprises a fructose unit. Fructose may then be provided from a fructogenic compound by enzymatic activity in the body, for example.

In one embodiment of the present invention, the carbohydrate fraction of the nutritional composition comprises at least 30 % glucose and fructose, preferably 50 % glucose and fructose more preferably at least 85 % glucose and fructose. In one embodiment of the present invention the carbohydrate fraction of the nutritional composition provides at least 50 %, preferably at least 70 % of the energy of the bar.

The sugar substitute may be any sugar substitute that is known in the art and suitable for human and/or animal consumption.

For example, the sugar substitute may be selected from the group consisting of natural sugar substitutes and artificial sugar substitutes or combinations thereof.

Natural sugar substitutes may be for example Brazzein, Curculin, Erythritol, Fructose, Glycyrrhizin, Glycerol, Hydrogenated starch hydrolysates, Lactitol, Lo Han Guo, Mabinlin, Maltitol, Maltooligosaccharide Mannitol, Miraculin, Neoculin, Monellin, Pentadin, Sorbitol, Stevia, Tagatose, Thaumatin, and/or Xylitol or combinations thereof.

Artificial sugar substitutes may be, for example, Acesulfame potassium, Alitame, Aspartame, salts of aspartame-acesulfame, Cyclamate, Dulcin, Glucin, Isomalt, Neohesperidin dihydrochalcone, Neotame, P-4000, Saccharin, SC45647, and/or Sucralose, or combinations thereof.

Generally, the sugar substitutes are used in amounts that correspond to their perceived sweetness with respect to sugar. For example a part of the sugar, such as the fructose glucose mixture, may be replaced by sugar substitutes in an amount that the resulting sweetness imparted by the sugar substitute(s) is in the range of ½ of the sweetness of the replaced sugar to 2x the sweetness of the replaced sugar.

Typically the sweetness of the sugar substitutes is perceived as follows:

| **Sugar substitute** | **Sweetness by weight compared to sugar** |
|---|---|
| Brazzein | 800x |
| Curculin | 550× |
| Erythritol | 0.7x |
| Glycyrrhizin | 50× |
| Glycerol | 0.6x |
| Hydrogenated starch hydrolysates | 0.4×-0.9× |
| Lactitol | 0.4x |
| Lo Han Guo | 300x |
| Mabinlin Protein | 100× |
| Maltitol | 0.9× |
| Mannitol | 0.5× |
| Monellin | 3000x |
| Pentadin | 500× |
| Sorbitol | 0.6x |
| Stevia | 250x |
| Tagatose | 0.92x |
| Thaumatin | 2000x |
| Xylitol | 1.0× |
| Acesulfame potassium | 200x |
| Alitame | 2,000x |
| Aspartame | 160-200× |
| Salt of aspartame-acesulfame | 350x |
| Cyclamate | 30x |
| Dulcin | 250x |
| Glucin | 300x |
| Neohesperidin dihydrochalcone | 1500× |
| Neotame | 8000x |
| P-4000 | 4000x |
| Saccharin | 300x |
| Sucralose | 600x |
| Isomalt | 0.45×-0.65× |

In general, however, the sugar substitute is used in the nutritional composition in accordance with the present invention in a weight ratio of 1:1 to 1:1000000, preferably 1:1 to 1: 100000, even more preferred 1:1 to 1: 10000, most preferred 1:1 to 1:1000 compared to the total carbohydrate fraction.

The sugar substitute may be used in the nutritional composition in accordance with the present invention in a weight ratio of 1:1 to 1:1000000, for example 1:10 to 1: 100000, 1:100 to 1: 10000, or 1:500 to 1:2000 compared to the total amount of glucose and fructose.

The carbohydrate fraction of the nutritional composition may comprise dextrose, fructose and/or maltodextrins.

The nutritional composition comprises a carbohydrate fraction and, optionally, a protein fraction and/or a fat fraction.

The presence of proteins and/or fats in the composition of the present invention has the advantage that this way it is possible to provide the athlete with a more complete nutrition during performance. Furthermore, the presence of proteins allows producing a product with an improved taste.

As protein source, any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred.

The proteins may be intact or hydrolysed or a mixture of intact and hydrolysed proteins. It may be desirable to supply partially hydrolysed proteins (degree of hydrolysis between 2 and 20%), for example for athletes believed to be at risk of developing cows' milk allergy. Additionally, in general at least partially hydrolysed proteins are easier and faster to metabolize by the body. This is in particular true for amino acids. Consequently, it is further preferred if the nutritional composition contains single amino acids, most preferred are essential amino acids. In one embodiment the composition contains amino acids such as L-leucine, L-valine and/or L-isoleucine.

If the composition includes a fat source, the fat source has the advantage that for example an improved mouth feel can be achieved. Any fat source is suitable. For example animal or plant fats may be used. To increase the nutritional value, n3-unsaturated and n6-unsaturated fatty acids may be comprised by the fat source. The fat source may also contain long chain fatty acids and/or medium chain fatty acids. For example, milk fat, canola oil, corn oil and/or high-oleic acid sunflower oil may be used.

The nutritional composition may be formulated in a way that it contains less than 40 g protein per 100g and/or less than 20 g fat per 100g.

It may also contain minerals and micronutrients such as trace elements and vitamins in accordance with the recommendations of Government bodies such as the USRDA

The composition of the present invention may contain vitamins, such as Vitamin C, Vitamin E, Vitamin B12, Niacin, Vitamin B6, folic acid, biotin, panthotenic acid, Vitamin B₂ and/or Vitamin B6, preferably in amounts that correspond to at least 10 % of the recommended daily dose.

The presence of vitamins may contribute to the effectiveness of the product and may further protect the athlete. For example, the presence of vitamin C will help to protect against catching a common cold.

The composition may also comprise electrolytes and/or minerals, such as sodium, potassium, calcium, iron, magnesium or zinc.

These compounds may be helpful to replenish the body with compounds that he is constantly loosing due to the generation of sweat during exercise. They may also help to avoid the generation of sore muscles.

The nutritional composition may further contain one or more compounds selected from the group consisting of aroma compounds, fibre, caffeine, conservatives, guarana, acidifying agents, binding agents, gel building material, water, fruit juice, fruits, antioxidants, colouring agents.

These agents may improve the product of the present invention with respect to many properties, such as taste, consistency, colour, stability during storage, digestibility, and many more that are known to those of skill in the art.

The energy density of the composition is not critical for its effectiveness. However, a high energy density has the advantage that less food needs to be ingested to replenish carbohydrates as fuel to the body. Consequently, high energy densities are preferred for the composition of the present invention.

Obviously, however, drinks will have a much lower energy density than other food products such as bars or gels.

If the nutritional composition of the present invention is a solid food product, such as, e.g., a carbohydrate bar, it may have an energy density of 800-2200 kJ/100g, preferably 1000-2000 kJ/100g, most preferred 1200-1800kJ/100g.

To be easily consumable - for example during a competition or in between competitions - the serving size of a solid nutritional composition of the present invention is preferably relatively small. Preferably, such a dry product has a serving size of 10-200 g, preferably 20-100g, most preferred 50-80g. Alternatively, it may also be provided as bite size bars with a serving size between 3 and 15g, preferably between 5 and 10g. This way, the carbohydrate uptake can precisely be adjusted to the needs of an athlete.

If the nutritional composition of the present invention is a liquid or gel-like food product, such as, e.g., a drink, it may have an energy density of 50-1500 kJ/100ml, preferably 75-1300 kJ/100ml, most preferred 80-1000kJ/100ml. The serving size of such drinks may be in the range of 50ml-500ml, for example 100ml-350 ml. The serving size of a gel may be in the range of 20-100ml, for example 30-50 ml.

A composition of the present invention may comprise the following percentages of daily values (DV) based on a 2000 calorie diet: Between 4 and 6 % total fat, including between 1 and 3 % saturated fats, between 5 and 9 % sodium, between 0,5 and 1,5% potassium, between 12 and 16 % carbohydrates, including between 5 and 10 % glucose and fructose, and between 10 and 14 % proteins.

Additionally it may comprise between 80 and 120 % DV vitamin C, between 20 and 30 % DV calcium, between 25 and 35 % DV iron, between 80 and 120 % DV vitamin E, between 80 and 120 % DV thiamin, between 80 and 120 % DV riboflavin, between 80 and 120 % DV niacin, between 80 and 120 % DV vitamin B6, between 80 and 120 % DV folate, between 80 and 120 % DV vitamin B12, between 80 and 120 % DV biotin, between 80 and 120 % DV pantothenic acid, between 20 and 30 % DV phosphorus, between 20 and 30 % DV magnesium, between 25 and 35 % DV zinc, between 25 and 35 % copper, and between 15 and 25 % DV chromium.

The nutritional composition in accordance with the present invention may further comprise one or more compounds selected from the group consisting of aroma compounds, fiber, caffeine, conservatives, guarana, acidifying agents, binding agents, gel building material, water, fruit juice, fruits, antioxidants, colouring agents.

The present invention also relates to the use of a sugar substitute for the preparation of a composition to provide an increased performance, in particular endurance performance.

The composition may be a nutritional composition as described in one or more of the embodiments described above.

The composition may also be used to treat or prevent a lack of performance, in particular endurance performance

The uses described in the present invention may be medical uses or non-medical uses.

The composition prepared by the use of the present invention may also be used to provide an increased gastrointestinal tolerance and/or to treat or prevent problems with the gastrointestinal tract. Also the gastrointestinal tolerance for carbohydrates, in particular for the glucose/fructose mixture of the present invention will be increased.

The problems with the gastrointestinal tract as mentioned above are not particularly limited but are preferably selected from the group consisting of upper abdominal problems such as reflux, heartburn, bloating, upper abdominal cramps, vomiting, nausea; lower abdominal problems such as intestinal cramps, flatulence, urge to defecate, left abdominal pain, right abdominal pain, loose stool, diarrhoea; or systemic problems such as dizziness, headache, muscle cramp or urge to urinate.

For exercise in general, but in particular for competitive exercise it is essential that the body has blood sugar available for the muscles to burn at all times. In particular at the end of a race it must be avoided that an athlete runs out of energy. The subject matter of the present invention is well suited to prevent this. According to one embodiment of the present invention the composition of the present invention can be used to allow for enhanced blood sugar maintenance late in exercise and/or an increased exogenous CHO oxidation rate at exercise onset.

The composition of the present invention can not only be used to secure long lasting blood sugar maintenance, it can also be used to provide an increased exogenous carbohydrate oxidation. This way, the energy delivery from carbohydrates during physical exercise can be maximised.

Further, composition may be used to provide faster energy delivery, in particular to working muscles, and/or to provide more sustained energy to muscles. Both effects will contribute to an optimal performance of an athlete.

Finally, the composition may also be used to treat or prevent symptoms of fatigue and/or to improve cycling cadence, for example measured in revolutions per minute and/or to decrease ratings of perceived exertion (RPE).

The composition of the present invention may ideally be used in an amount that corresponds to an ingestion of at least 30g CHO/h, preferably at least 50g CHO/h, more preferably at least 65g CHO/h and most preferably between 80g CHO/h and 110g CHO/h.

The inventors have found that the higher the amount of carbohydrates ingested per hour is, the more the exogenous carbohydrate oxidation can be increased.

The composition prepared by the use of the present invention may also be used to up-regulate the expression of intestinal SGLT1 and GLUT-5 glucose and fructose transporters. This way an optimal absorption of carbohydrates, in particular of glucose and fructose, from food sources can be established.

It is clear to those skilled in the art that they can freely combine all features of the present invention disclosed herein without departing from the subject matter as disclosed. It particular features described with respect to the nutritional composition of the present invention and with respect to the uses of the present invention may be combined.

Further features and advantages of the present invention are apparent from the following Examples.

### Example 1: Carbohydrate gel

Carbohydrate gels of the present invention can be prepared by any method known in the art.

For example, gels can be prepared by a method comprising the following three steps. Step one involves the mixing of the ingredients and adding all the ingredients to the cooker. The primary purpose of this step is to have a smooth and homogenous mass, which is partially achieved though good stirring so all ingredients are properly dissolved. Step two involves the heating of the mass to a temperature of 75°C for at least 10 minutes. The primary purpose of step two, which is the heating process, is to have a microbiological control and to lower the viscosity of the product to facilitate the filling process. Step three is the filling process, which is the hot-filling of the gel into pouches, followed by the hot-sealing of the pouches.

A typical gel formulation may comprise:
A carbohydrate blend (maltodextrin, fructose and/or glucose) in combination with Aspartame in a weight ratio of 800:1 , filtered water, electrolyte blend (sodium chloride, sodium citrate, potassium chloride), citric acid, natural flavours, sodium benzoate, potassium sorbate, L-leucin (170mg/100g), L-valin (170mg/100g), L-isoleucin (170mg/100g), Vitamin A and Vitamin C.

### Example 2: Bar formulation

A typical formulation for a bar in accordance with the present invention is presented in table form below:

| **Ingredient** | **Amount (weight-%)** |
|---|---|
| Maltodextrin | 12.917% |
| Aspartame | 0.016% |
| Milk Protein Isolate | 8.979% |
| Soy Protein Isolate | 0.500% |
| Rice Flour | 5.273% |
| Flour Peanut | 0.100% |
| Oat Bran | 14.360% |
| Rice Crisps | 8.985% |
| Crystalline Fructose | 6.800% |
| Evap Cane Juice Syrup | 33.150% |
| salt | 0.300% |
| Glycerine, | 1.000% |
| Almond Butter | 3.278% |
| Vanilla | 1.234% |
| Vitamin/Mineral/Amino Premix | 3.108% |
| **Total** | **100.000%** |

The bar is produced as follows:
All wet ingredients are mixed together (syrup, glycerine, almond butter and flavours) at 50°C. Separetaly, dry ingredients are mixed together, then the wet slurry is added to the dry mix and the mass is mixed for 2 to 5 minutes under high shear. The dough is slabed and cut into bar shape before packing.

## Claims

1. Nutritional composition comprising a carbohydrate fraction containing glucose and fructose in a ratio in the range of 3:1 to 1:1, and at least one sugar substitute.

2. Nutritional composition in accordance with claim 1, **characterized in that** the carbohydrate fraction comprises at least 30 % glucose and fructose, preferably 50 % glucose and fructose more preferably at least 85 % glucose and fructose.

3. Nutritional composition in accordance with one of the preceding claims, wherein the sugar substitute is selected from the group consisting of natural sugar substitutes, for example Brazzein, Curculin, Erythritol, Fructose, Glycyrrhizin, Glycerol, Hydrogenated starch hydrolysates, Lactitol, Lo Han Guo, Mabinlin, Maltitol, Maltooligosaccharide Mannitol, Miraculin, Monellin, Neoculin, Pentadin, Sorbitol, Stevia, Tagatose, Thaumatin, and/or Xylitol, and artificial sugar substitutes, for example Acesulfame potassium, Alitame, Aspartame, salts of aspartame-acesulfame, Cyclamate, Dulcin, Glucin, Isomalt, Neohesperidin dihydrochalcone, Neotame, P-4000, Saccharin, SC45647 and/or Sucralose.

4. Nutritional composition in accordance with one of the preceding claims, wherein the sugar substitute is used in a weight ratio of 1:1 to 1:1000000 , preferably 1:1 to 1:100000, even more preferred 1:1 to 1:10000, most preferred 1:1 to 1:1000, compared to the total carbohydrate fraction present in the composition

5. Nutritional composition in accordance with one of the preceding claims, **characterized in that** it further contains vitamins, such as Vitamin C, Vitamin E, Vitamin B12, Niacin, Vitamin B6, folic acid, biotin, panthotenic acid, Vitamin B₂ and/or Vitamin B6, preferably in amounts that correspond to at least 10 % of the recommended daily dose.

6. Nutritional composition in accordance with one of the preceding claims, **characterized in that** it further contains amino acids such as I-leucine, L-valine and/or L-isoleucine.

7. Use of a sugar substitute for the preparation of a composition to provide an increased CHO absorption, oxidation, and/or performance, in particular endurance performance.

8. Use in accordance to claim 7, wherein the composition is a nutritional composition in accordance with one of claims 1-6.

9. Use in accordance with one of claims 7-8, to provide an increased gastrointestinal tolerance and/or to treat or prevent problems with the gastrointestinal tract.

10. Use in accordance with claim 9, wherein the problems with the gastrointestinal tract are selected from the group consisting of upper abdominal problems such as reflux, heartburn, bloating, upper abdominal cramps, vomiting, nausea; lower abdominal problems such as intestinal cramps, flatulence, urge to defecate, left abdominal pain, right abdominal pain, loose stool, diarrhoea; or systemic problems such as dizziness, headache, muscle cramp or urge to urinate.

11. Use in accordance with one of claims 7-10 to allow for an enhanced blood sugar maintenance late in exercise.

12. Use in accordance with one of claims 7-11 to provide an increased exogenous carbohydrate oxidation, to provide faster energy delivery, in particular to working muscles, and/or to provide more sustained energy to muscles.

13. Use in accordance with one of claims 7-12 to further treat or prevent symptoms of fatigue, to improve cycling cadence, for example measured in revolutions per minute and/or to decrease ratings of perceived exertion (RPE).

14. Use in accordance with one of claims 7-13, wherein the composition is used in an amount that corresponds to an ingestion of at least 30g CHO/h, preferably at least 50g CHO/h, more preferably at least 65g CHO/h and most preferably between 80g CHO/h and 110g CHO/h.
